# EUROPEAN PATENT APPLICATION

(11) **EP 1 632 196 A1**
(43) Date of publication of application: **08.03.2006**
(21) Application number: 05255442.5
(22) Date of filing: 06.09.2005
(51) Int. Cl.: A61B 19/04, B65D 83/08

(54) **Mountable glove dispenser**

(30) Priority: 07.09.2004 US 607744 P
(71) Applicant: Foodhandler Inc., Westbury, 11590 New York (US)
(72) Inventor: West, Kimberly Ann, Reno Nevada 89523 (US)
(74) Representative: JENSEN & SON

(57) **Abstract**

Provided is an apparatus for dispensing gloves, which includes a container 110 having front 120 and rear surfaces 130, with a dispenser opening 115 in the front surface. A stack of glove elements 510 is disposed within the container, with the individual glove elements being attached to each other by perforation lines 530 or other lines of weakness. In some implementations, the stack of glove elements 510 is attached to an insert 570 within the container. Preferably, the apparatus includes a mounting hole 190 or other means for attaching the rear surface of the container to a wall.

## Description

Priority is claimed to United States Provisional Patent Application Serial No. 60/607,744, filed on September 7, 2004, and titled "Wall Mounted Individual Glove Dispenser", which application is incorporated by reference herein as though set forth herein in full.

The present invention pertains to a glove dispenser for dispensing disposable gloves that can be mounted on a wall or on a separate stand or other apparatus.

Disposable gloves are mandatory equipment in many industries that require clean environments, such as the food-service industry. The use of disposable gloves often reduces the spread of viruses and other contaminants among individuals.

Many types of disposable gloves are known to and used by the food-service industry. One type comprises two superimposed layers of thermoplastic film sealed together along their peripheries, leaving an opening for a hand to be inserted therein. A two-layered disposable mitt is shown in Grinberg U.S. Pat. No. 5,806,099, and a method of forming such a mitt is shown in Bradfield U.S. Pat. No. 4,928,322.

These disposable gloves are typically fabricated using vinyl, latex or polyethylene. Disposable gloves are generally sold in stacked units comprising a supply of gloves layered one on top of another. Gloves may be sold in a dispenser, such as a paperboard box, which encloses the stack and from which gloves may be removed one at a time. A box dispenser thus provides a simple and economical means for protecting the stored gloves from contamination and for dispensing the gloves. Variations of box dispensers for disposable gloves are shown in U.S. Patent No. 4,844,293 to McLaughlin and U.S. Patent No. 5,655,682 to Hoffrichter.

Box dispensers have several drawbacks, however. For example, a disposable glove is often difficult to don after removing it from a box dispenser. Upon being dispensed, the glove may have creases and/or be folded, requiring a user to straighten it out before donning it. A user may have difficulty first finding and then separating the glove opening into which the hand is inserted. Therefore, additional dispensing configurations have also been developed. One such configuration comprises a stack of disposable gloves held together by a heat-fused detachable portion of the gloves, which portion may be covered by a flap as shown in Kiecena U.S. Pat. No. 5,966,741. The gloves advantageously remain flat as they are removed from the detachable portion, in order to facilitate the process of donning them after they are removed.

Conventionally, dispensing assemblies include a dispensing box or a saddle or rack for mounting the gloves. By providing a free-standing or a rack dispenser, the placement of the dispenser itself may tend to interfere with food preparation, and the chance is increased of contamination of the dispenser itself with the food being prepared.

The present inventor has recognized that contaminants are less likely to be transmitted to food being handled by an employee when that employee regularly wears gloves, and regularly replaces used gloves with fresh ones. The gloves, therefore, should be easily accessible, and should require little handling to be donned.

The present invention addresses this problem by providing an apparatus for dispensing gloves, which includes a container having front and rear surfaces, with a dispenser opening in the front surface. A stack of glove elements is disposed within the container, with the individual glove elements being attached to each other by perforation lines or other lines of weakness. In some implementations, the stack of glove elements is attached to an insert (such as a tray) within the container. Preferably, the apparatus includes a mounting hole or other means for attaching the rear surface of the container to a wall.

In a representative embodiment, a wall-mounted or wall-mountable glove dispenser for facilitating individual glove dispensing is provided. The dispenser has walls that define a cavity and an opening thereof. A plurality of gloves is disposed within the dispenser. The dispenser may be fabricated from a flat sheet of material folded to define the cavity. Provision is made in the design of the flat sheet for a tab extending away from the cavity and having a mounting opening therein to allow the dispenser to be mounted on a wall or other vertical surface. A disposable glove may be donned easily and quickly from the present dispenser, thus encouraging the frequent and regular replacement of used gloves with new ones.

The present invention, in representative embodiments, uses a wall-mounted or wall-mountable glove dispenser for facilitating individual glove dispensing as opposed to a rack-mounted or free-sitting glove dispenser. The present invention also provides an improved configuration for a disposable glove system whereby a disposable glove may be donned more easily and quickly than heretofore, in order to encourage the frequent and regular replacement of used gloves with new ones. The new and improved system of the present invention is especially valuable in the food-service industry, where efficiencies in the nature of timesaving and in the ease and simplicity of donning the gloves can result in significantly increased productivity and enhanced hygiene.

In an exemplary embodiment of the present invention, a glove dispenser is provided. The dispenser has walls that define a cavity and an opening thereof. A plurality of gloves is disposed within the dispenser. The dispenser may be fabricated from a fiat sheet of material folded to define the cavity. Provision is made in the design of the flat sheet for a tab extending away from the cavity and having a mounting opening therein to allow the dispenser to be mounted on a wall or other vertical surface.

In another exemplary embodiment of the present invention, a method of packaging gloves in a glove dispensing package is provided. A sheet of material is formed having creases or folding axes thereon. The sheet of material may be folded along these axes to define the cavity enclosing one or more gloves. The folding procedure also includes the formation of a tab extending from the cavity and having a mounting opening therein to allow the dispenser to be mounted on a wall or other vertical surface.

The first layer of the glove may be provided with one or more mounting openings and matching lines of weakness extending from the mounting openings to the rear edge of the first layer. The first layer may also be provided with one or more mounting openings near the rear edge of the first layer, separated from the remainder of the first layer by a transverse line of weakness. In either case, a plurality of gloves may be mounted in the present dispenser using a fastener inserted through the mounting opening(s) of the gloves to attach them to a tray or other insert. This insert may then be placed inside the present dispenser, allowing the gloves to be withdrawn through an opening in the dispenser.

Gloves may be removed from the dispenser by grasping a potion of the glove and/or partially inserting a hand into the glove and applying a force to the glove in a direction of donning. As a result, the glove can be separated from its mounting means either by tearing along the transverse line of weakness or along the matching lines of weakness extending from the mounting openings to the rear edge of the first layer. The first and second layers of the glove may be fabricated from a material having little elasticity, thereby causing the opening to open with little effort by the user.

The foregoing summary is intended merely to provide a brief description of the general nature of the invention. A more complete understanding of the invention can be obtained by referring to the claims and the following detailed description of the preferred embodiments in Connection with the accompanying figures.
FIG. 1 is a top plan view of an unassembled dispenser container according to a representative embodiment of the present invention.
FIG. 2 shows a front perspective view of a glove contained within a dispenser according to a representative embodiment of the present invention.
FIG. 3 shows an attached stack of glove elements that can be contained within a dispenser according to a representative embodiment of the present invention.
FIG, 4 shows an exploded view of a dispenser container enclosing a stack of glove elements attached to a tray insert, according to the preferred embodiment of the present invention.
FIG. 5 is a perspective view of a closed and assembled dispenser, in use, according to a representative embodiment of the present invention.

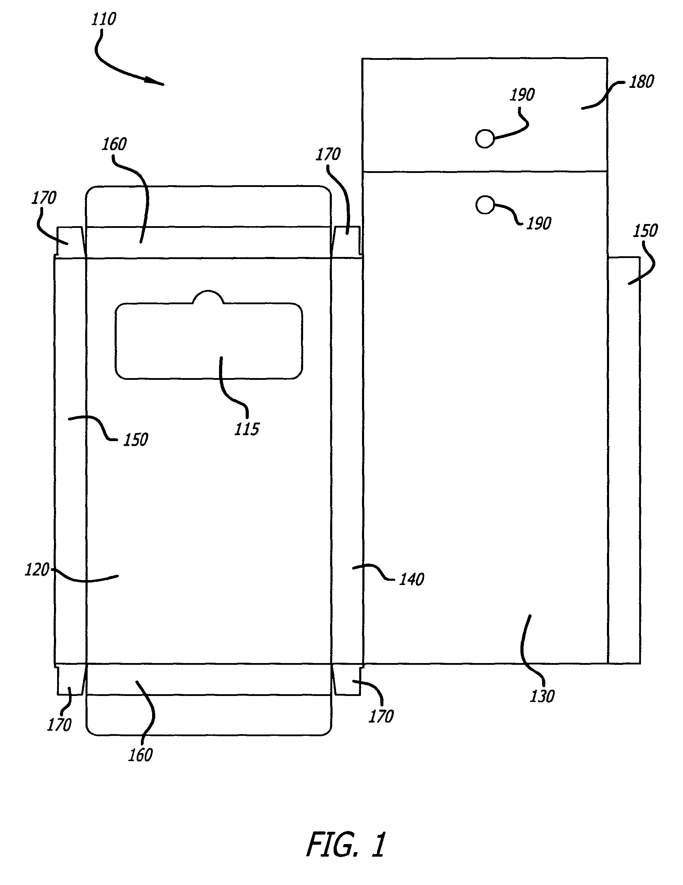

FIG. 1 is a top plan view of an unfolded dispenser Container 110 according to an exemplary embodiment of the present invention. The dispenser container 110 includes a front panel 120 and a back panel 130 connected by a spine 140. In alternative embodiments, the dispenser container 110 may be formed from a single sheet of paperboard, chipboard, corrugated cardboard or other suitable material. Preformed creases may be used to separate various areas of the dispenser container 110, such as the aforementioned front panel 120, back panel 130 and the spine 140. As such, the container may be folded in upon itself to form a box capable of holding a supply of gloves to be dispensed.

The dispenser container 110 preferably also includes a pair of vertical flaps 150 and a pair of horizontal flaps 160. These flaps aid in the conversion of the dispenser container into an enclosed box. In one embodiment, the vertical flaps 150 may be laid over and attached to one another with an adhesive or fastening device. A number of tabs 170 that attach to the vertical flaps 150 and/or to the spine 140 allow the horizontal flaps 160 to be tucked into the top and bottom openings of the dispenser container 110, thus enclosing the fully assembled box shape of the dispenser container 110.

The dispenser container 110 may be punched or cut out of a single flat sheet of material, or it may be fabricated from multiple individual sheets which are themselves laminated together to form a single dispenser container 110. The container may be fashioned with preformed creases to separate the various panels, or it may be provided with perforations, allowing a user or producer of the container to form the creases upon construction of the enclosed box from the flat piece. In one embodiment, the dispenser container 110 is made of cardboard and is laminated on one or both sides with a water barrier, such as plastic, to further protect the gloves contained within from contamination.

The dispenser container 110 includes a dispenser opening 115 allowing the supply of gloves within the assembly to be dispensed one by one through the dispenser opening 115. The container also includes an extended back panel 130 which, along with a reinforcing panel 180, may be folded together to form a reinforced tab extending for a greater length than the front panel 120 of the enclosed box when the dispenser container is assembled into an enclosed box. This reinforced tab, along with a mounting opening 190, allows the dispenser container 110 to be easily mounted on a wall or other surface.

By mounting the dispenser container 110 on a wall or other such vertical surface away from any work surfaces where food is being prepared, allowance is made for an economical use of space. In addition, the danger of contamination of the glove supply by food or other contaminants is lessened. In an alternative embodiment, the reinforcing panel 180, together with the reinforcing tab, may be provided with alternative fastening means to allow the dispenser to be positioned on a wall or other vertical surface. An adhesive may also be provided on the back panel 130 to allow the dispenser container 110 to adhere directly to a wall.

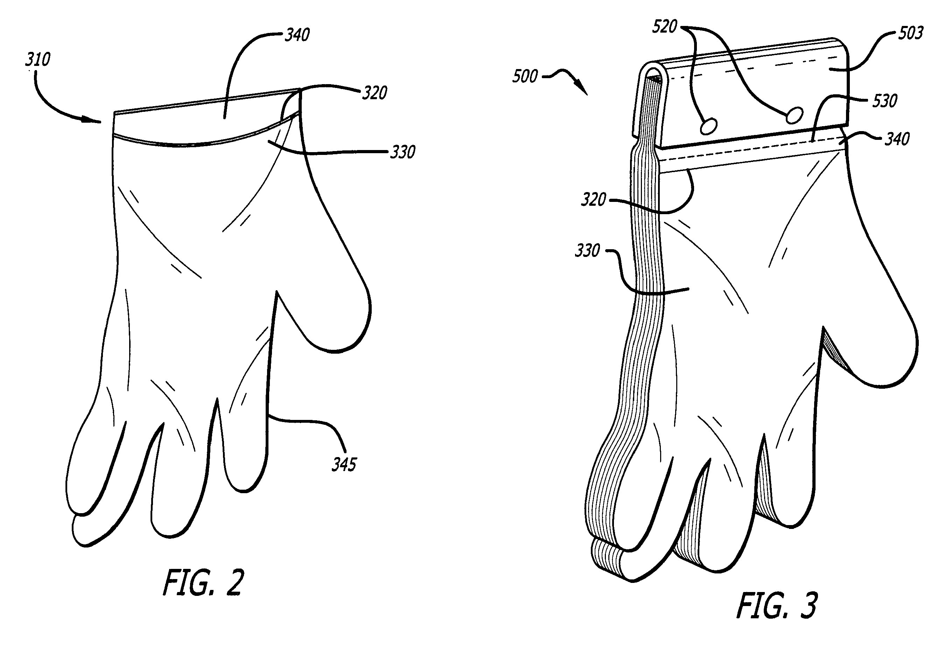

FIG. 2 shows a glove 310 for use in the present assembly. The glove may be made of vinyl, latex, polyethylene, or any other suitable material. The glove 310 has a front sheet 330 and a back sheet 340. A demarcation line 320 denotes the edge of the sheet nearer to the user for the top glove 310 in the dispenser container 110. The front and back sheets are joined together along their peripheries, save along the demarcation line 320 (which is the open seam into which the users hand is inserted). The front sheet 330 is advantageously shorter than the back sheet 340 to provide a bare inner surface of the back sheet 340 along which a hand may be slid easily into the interior of the glove 310. The front and back sheets are superimposed and preferably are fabricated from tear-resistant plastic film, such as polyethylene film. This film has the additional advantage of having little elasticity, causing the glove 310 to open immediately with little effort by a user upon donning the glove. The front and back sheets may be joined by heat welding or a similar process along the outer edge 345.

The glove 310 may be non-transparent, transparent or semi-transparent, and may have any desired color. The glove 310 may have all five fingers, as shown in FIG. 2, or may be a mitt having a single compartment for four fingers and a separate thumb compartment. The glove 310 may also be provided in various different sizes. A supply of multiple gloves in a single dispenser container 110 may be provided in a range of sizes within a single assembly, or all in the same size.

FIG. 3 shows a glove assembly 500 or "saddle" comprising a stack of multiple glove elements for use in the present dispenser. The back sheet 340 of each glove has a perforation line 530 substantially perpendicular to the longitudinal axis of the glove. Above this perforation line 530 is at least one mounting opening 520 for receiving a fastener. In a preferred embodiment, two or more mounting openings 520 are used to secure the glove assembly 500 within the present dispenser.

Alternatively, the back sheet 340 of each glove may have longitudinal lines of weakness (not shown) extending from each mounting opening 520 to the rearmost edge of the back sheet 340. Such lines of weakness, substantially parallel to the longitudinal axis of the glove, provide an alternative axis along which an individual glove may be removed from the glove assembly 500. Longitudinal lines of weakness replace the perforation line 530, thus eliminating any residual portion of an individual glove that might otherwise remain on the glove assembly 500 after the glove is removed.

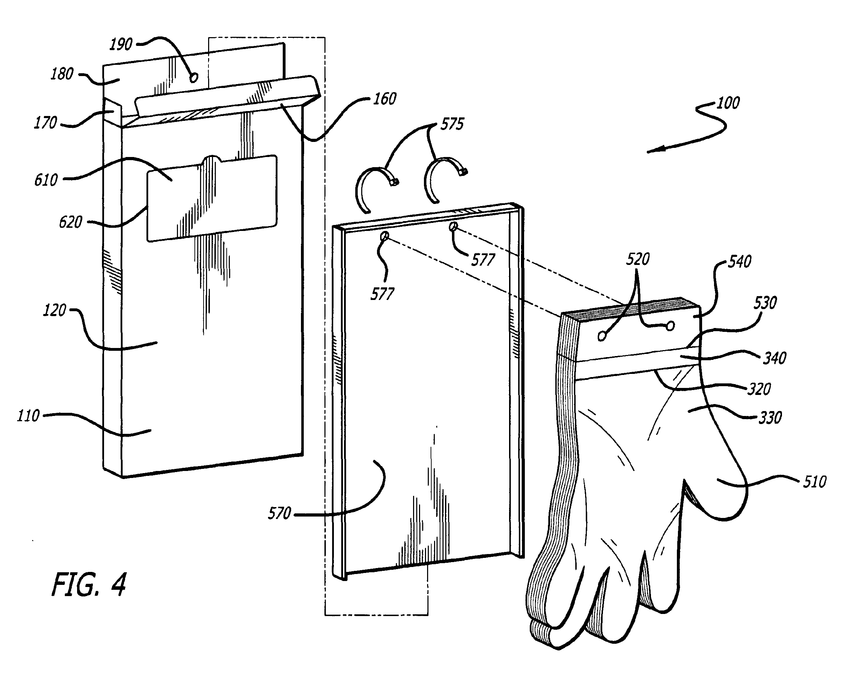

The glove assembly 500 may comprise an integral stack of individual gloves heat welded together in one or more regions of their back sheets 340. Alternately, the individual gloves may be fastened to each other by tie straps extending through their mounting openings 520. These tie straps and/or the fasteners may also bind the glove assembly 500 to a tray or other insert (e.g., as shown in FIG. 4) immediately beneath the glove assembly 500 in order to hold the assembly securely thereto. This insert may then be placed inside the present dispenser, allowing the gloves to be withdrawn through the dispenser opening 115 as needed.

In the embodiment shown in FIG. 3, the gloves are held together using fasteners (not shown) that are inserted through holes 520 and through matching holes in a sturdier more shape-retaining element 503. For example, element 503 may be formed of cardboard.

Although the gloves of the glove assembly 500 shown in FIG. 3 may be worn on either the right or left hand, they are more easily donned by the right hand. The glove may also be donned by the left hand by rotating turning one's left hand palm-up while donning the glove. A stacked unit of left-handed gloves comprising a glove assembly 500 that is the mirror image of the assembly of FIG. 3, but otherwise identical, may also be manufactured to allow a user to don gloves on both hands in the more ergonomic palm-down manner.

FIG. 4 shows an exploded view of a dispenser container 110 with a stack of glove elements 510 attached to a tray insert 570 according to the preferred embodiment of the present invention. As shown, in the present embodiment dispenser container 110, when folded into its operative shape, resembles a box having a tab 180 extending from its top edge. In the present embodiment, a hole 190 functions as a means for attaching the rear surface of dispenser container 110 to a wall or other device. That is, hole 190 permits dispenser container 110 to be hung from a hook, nail or similar device.

In the present embodiment, a removable panel 610 covers the dispenser opening 115. Preferably, panel 610 is simply a part of the single sheet of material that is used to form the remainder of the container and is formed by perforating its periphery 620. Accordingly, removable piece 610 may be punched out by the user along the perforation line 620 and discarded. In this manner, gloves inside the assembly may be removed without making an unnecessarily large hole in the front panel 120. The shape and/or size of the dispenser opening 115 may be different in other embodiments.

In the illustrated preferred embodiment, tray 570 is slightly larger in width and length than a single glove element 510. In addition, tray 570 has holes 577 that match the holes 520 in the stack of glove elements 510. Accordingly, the stack of glove elements 510 is laid into tray 570 and then attached to tray 570 using cable ties or other fasteners 575. Thereafter, tray 570 is simply slid into container 110, e.g., through the top opening of container 110. Upon closing flap 160, the device is completed and ready for use.

The glove elements 510 are similar to those depicted in FIG. 3, with an upper section 540 when the glove portion is separated from the remainder of the glove element 510 by tearing along perforated line 530. Here, however, unlike assembly 500 shown in FIG. 3, element 503 is omitted in favor of tray 570.

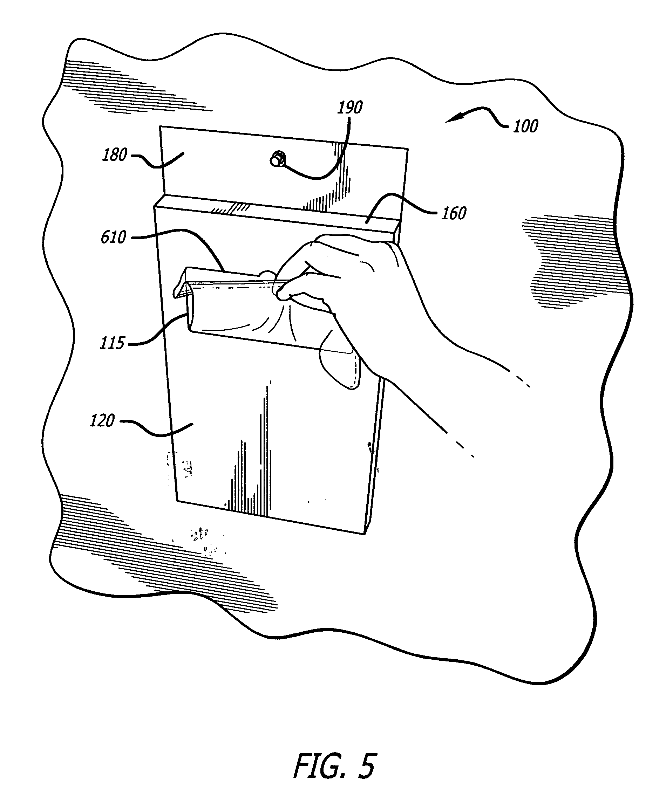

FIG. 5 shows a perspective view of an assembled dispenser 100, in use. The assembly 100 is shown here having the front panel 120, reinforced extending panel 180, dispenser opening 115 (e.g., after removing panel 610) and mounting opening 190.

A portion of the horizontal flap 160 is shown in FIG. 5, as are one or more gloves shown within the assembly 100. As noted above, the gloves preferably are formed from two flat sheets, one longer than the other.

The assembly 100 may have letters (e.g., stylized), pictures, logos, designs and/or other markings placed thereon through printing (e.g., multi-color), engraving, molding, or any other suitable method.

To don a glove, a user grasps a potion of the glove, preferably the edge of the front sheet 330 bounded by the demarcation line 320, and/or partially inserts a hand behind the front sheet 330 into the glove and applies a force to the glove in a direction of donning. This force causes either the perforation line 530 or, when present, the longitudinal lines of weakness to separate releasing an individual glove from the glove assembly 500. The glove may then be drawn out of the interior of the dispenser container 110 through the dispenser opening 115.

It will be appreciated by those of ordinary skill in the art that the invention can be embodied in other specific forms without departing from the spirit or essential character hereof. The present description is therefore considered in all respects to be illustrative and not restrictive, the scope of the invention to be determined by the appended claims and their equivalents.

For example, the dispensing assembly and the glove packaging method of the present invention may be applied to other disposable items such as stacks of food wrapping papers, food wrapping plastic sheets, or the like, that require sanitary packaging.

Several different embodiments of the present invention are described above, with each such embodiment described as including certain features. However, it is intended that the features described in connection with the discussion of any single embodiment are not limited to that embodiment but may be included and/or arranged in various combinations in any of the other embodiments as well, as will be understood by those skilled in the art.

Similarly, in the discussion above, functionality sometimes is ascribed to a particular module or component. However, functionality generally may be redistributed as desired among any different modules or components, in some cases completely obviating the need for a particular component or module and/or requiring the addition of new components or modules. The precise distribution of functionality preferably is made according to known engineering tradeoffs, with reference to the specific embodiment of the invention, as will be understood by those skilled in the art.

Thus, although the present invention has been described in detail with regard to the exemplary embodiments thereof and accompanying drawings, it should be apparent to those skilled in the art that various adaptations and modifications of the present invention may be accomplished without departing from the spirit and the scope of the invention. Accordingly, the invention is not limited to the precise embodiments shown in the drawings and described above. Rather, it is intended that all such variations not departing from the spirit of the invention be considered as within the scope thereof as limited solely by the claims appended hereto.

## Claims

1. An apparatus for dispensing gloves, comprising:
a container (110) having front and rear surfaces (120, 130), with a dispenser opening (115) in the front surface (120);
an insert (570) disposed within the container; and
a stack of glove elements (500), each glove element having an upper section that is attached to the insert and a glove attached to the upper section by a line of weakness (530).

2. An apparatus according to claim 1, further comprising attachment means (190) for attaching the rear surface of the container (110) to a wall.

3. An apparatus according to claim 2, wherein the attachment means comprises a tab extending away from the container (110) and having an opening for accommodating a mounting device.

4. An apparatus according to claim 1, wherein the gloves are comprised of plastic.

5. An apparatus according to claim 1, wherein the line of weakness is a perforated line.

6. An apparatus according to claim 1, wherein each glove is formed from two flat sheets, one longer than the other.

7. An apparatus according to claim 1, wherein the container (110) primarily is comprised of a paper product.

8. An apparatus according to claim 7, wherein the paper product is laminated with a water barrier.

9. An apparatus according to claim 7, wherein the container (110) is formed from a single sheet of the paper product, folded to form a cavity for holding the insert and the stack of glove elements.

10. An apparatus according to claim 1, wherein the container (110) primarily is comprised of a single sheet of folded material,

11. An apparatus according to claim 10, further comprising attachment means for attaching the rear surface of the container (110) to a wall.

12. An apparatus according to claim 10, wherein the single sheet of folded material is laminated with a water barrier.

13. An apparatus for dispensing gloves, comprising:
a container (110) having front and rear surfaces, with a dispenser opening in the front surface;
a stack of glove elements disposed within the container (110) and attached to each other by lines of weakness; and
means for attaching the rear surface of the container (110) to a wall.

14. An apparatus according to claim 13, wherein the attachment means comprises a tab extending away from the container (110) and having an opening for accommodating a mounting device.

15. An apparatus according to claim 13, wherein the container (110) primarily is comprised of a paper product.

16. An apparatus according to claim 15, wherein the paper product is laminated with a water barrier.

17. An apparatus according to claim 15, wherein the container (110) is formed from a single sheet of the paper product, folded to form a cavity for holding the stack of glove elements.

18. An apparatus according to claim 13, wherein the lines of weakness are perforated lines.

19. An apparatus according to claim 13, wherein the stack of gloves is attached to an insert within the container (110).

20. An apparatus according to claim 17, wherein the insert comprises a tray.
